# EUROPEAN PATENT SPECIFICATION

(11) **EP 0 308 336 B1**
(45) Date of publication and mention of the grant of the patent: **22.09.1993**
(21) Application number: 88402332.6
(22) Date of filing: 15.09.1988
(51) Int. Cl.: C12N 15/12, C12P 21/02

(54) **Process for the production of human pro-apolipoprotein A-I-like proteins**
Verfahren zur Herstellung von menschlichen pro-apolipoprotein-A-I-ähnlichen Proteinen
Méthode de production de protéines analogues de la pro-apolipoprotéine AI humaine

(30) Priority: 18.09.1987 JP 233898/87
(43) Date of publication of application: 22.03.1989
(73) Proprietor: MITSUBISHI KASEI CORPORATION, Tokyo 100 (JP)
(72) Inventor: Shibui, Tatsurou, Machida-shi Tokyo (JP); Kamizono, Michiru, Machida-shi Tokyo (JP); Teranishi, Yutaka, Sagimihara-shi Kanagawa-ken (JP)
(74) Representative: Peaucelle, Chantal

(56) References cited:
- EP-A- 0 293 357
- PATENT ABSTRACTS OF JAPAN, vol. 10, no. 276 (C-373)[2332], 19th September 1976; JP-A-61 96 998 (MITSUBISHI CHEM. IND. LTD) 15-05-1986
- PROTEIN ENGINEERING, vol. 1, no. 3, June 1987, page 250, Oxford, GB; A. ISACCHI et al.: "Use of protein fusions to increase gene expression of recombinant apolipoprotein AI"
- PROCEEDINGS OF THE NATIONAL ACADEMY OF SCIENCE USA, vol. 81, January 1984, pages 66-70, Washington, DC, US; S.W. LAW et al.: "Nucleotide sequence and the encoded amino acids of human apolipoprotein A-I mRNA"

## Description

### FIELD OF THE INVENTION

This invention relates to a process for the production of human pro-apolipoprotein A-I or human pro-apolipoprotein A-I-like substances having physiological activities equivalent to those of human pro-apolipoprotein A-I (referred to hereinafter as human pro-apolipoprotein A-I-like proteins) by using DNA-recombinant technology.

### DESCRIPTION OF THE PRIOR ART

Lipids in plasma consist essentially of cholesterol, phospholipids, triglycerides and free fatty acids. These lipids except the last one are conjugated with proteins, so that these water-insoluble lipids may be solubilized in plasma. Such lipid-protein complexes, which are called lipoproteins, have various molecular weights depending on the proportion of the lipid to protein. Lipoproteins have globular structures which may be considered to be composed of nonpolar triglycerides or cholesterol esters as a core and polar phospholipids or free cholesterols forming a superficial layer in association with proteins.

Proteins contained in lipoproteins are called apolipoproteins, ten or more of which are known at present. Apolipoproteins are essential constituents of lipoproteins and play an important role in the metabolism of lipoproteins as well.

Apolipoprotein A-I is one of the known apolipoproteins. It is also known that apolipoprotein A-I may activate lecithin-cholesterol acyltransferase (LCAT) which transfers a fatty acid at β-position of lecithin to free cholesterol to form cholesterol ester.

Those who are deficient in a normal apolipoptrotein A-I often suffer from metabolic disorder, resulting in hyperlipidemia and even arteriosclerosis. The normal functions of the apolipoprotein A-I in such patients can be recovered by administrating the normal apolipoprotein A-I to them.

In biosynthesis, pre-pro-apolipoprotein A-I is initially produced in apolipoprotein A-I producing cells followed by secretion into blood stream by means of a pre-segment (signal peptide). In the secretion process, the pre-segment is removed out and the resulting pro-apolipoprotein A-I is now subjected to hydrolysis in the blood stream by an apolipoportein A-I specific protease so as to be converted into a mature apolipoprotein A-I.

Up to now, some processes for the production of the mature apolipoprotein A-I through genetic engineering have been reported, for example, the process using CHO cells (Journal of Biological Chemistry, 262, 4241 - 4247, 1987) and the process using E. coli (Japanese Patent Appliation Laying Open (KOKAI) No.61-96998).

However, the former is not suitable for a mass-production because it utilizes animal cells (CHO cells) and the latter has a disadvantage that an efficiency of the expression of a desired protein is low.

We have now succeeded in producing pro-apolipoprotein A-I or pro-apolipoprotein A-I-like proteins with an expression efficiency of about 10,000 times higher than that in a conventional manner in which the mature apolipoprotein A-I is expressed, by expressing DNA sequence encoding pro-apolipoprotein A-I, especially that in which DNA sequence encoding a propeptide has been modified into a particular one.

### SUMMARY OF THE INVENTION

This invention provides a process for the production of human pro-apolipoprotein A-I or human pro-apolipoprotein A-I-like substances having physiological properties equivalent to those of human pro-apolipoprotein A-I, comprising introducing by transformation into host cells an expression vector containing DNA sequence encoding human pro-apolipoprotein A-I or human pro-apolipoprotein A-I-like substances having physiological properties equivalent to those of human pro-apolipoprotein A-I, culturing the transformed host cells and collecting the proteins produced thereby, characterised in that the DNA sequence encoding the pro-portion of the human pro-apolipoprotein A-I or human pro-apolipoprotein-like substances comprises the sequence:

5'- CGA CAT TTT TGG CAA CAA -3'
3'- GCT GTA AAA ACC GTT GTT -5'

### BRIEF DESCRIPTION OF THE FIGURES

The invention will be fully described with reference to the attached Figures, in which:
Fig.1 represents a retriction map of phAI-6;
Fig.2 represents phAI-sp in which Pst I and Sal I sites are introduced 5' to the apolipoprotein A-I gene by site-specific mutation;
Fig.3 represents phAI-spB in which Pst I and Sal I sites are introduced 5' to the apolipoprotein A-I gene and BglII site is introduced 3'to said same gene by site-specific mutation;

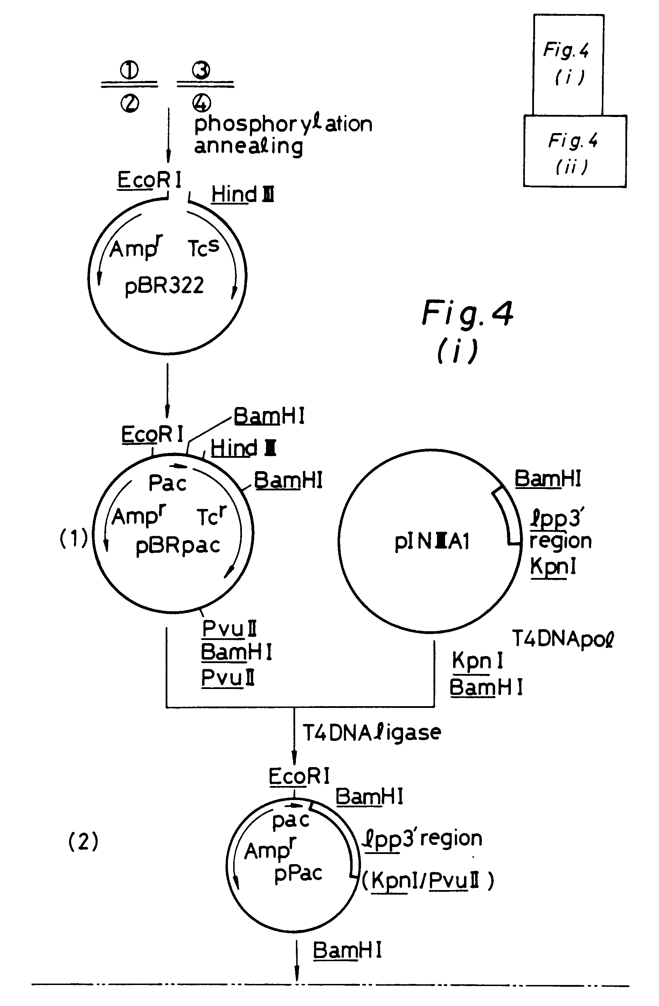

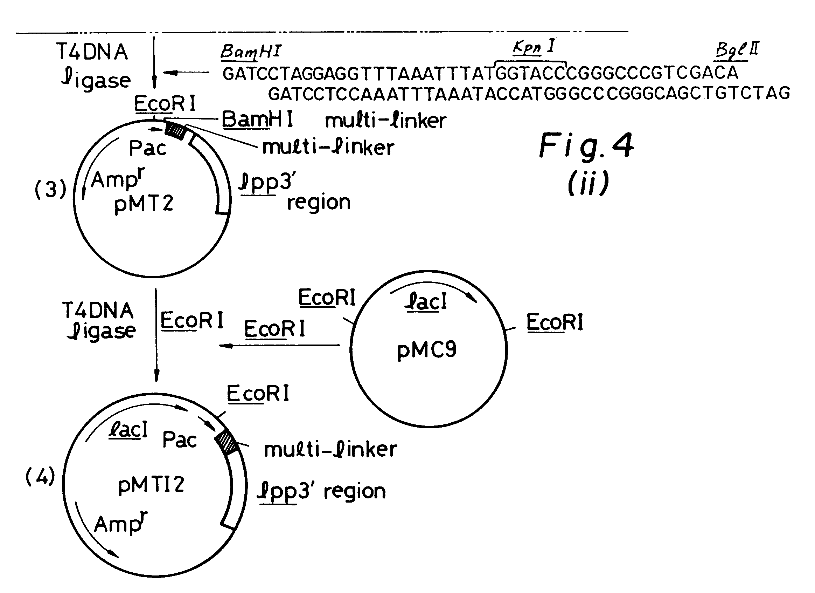

Fig.4 represents a scheme of the construction of an expression vector pMTI2 using a hybrid promoter (pac).

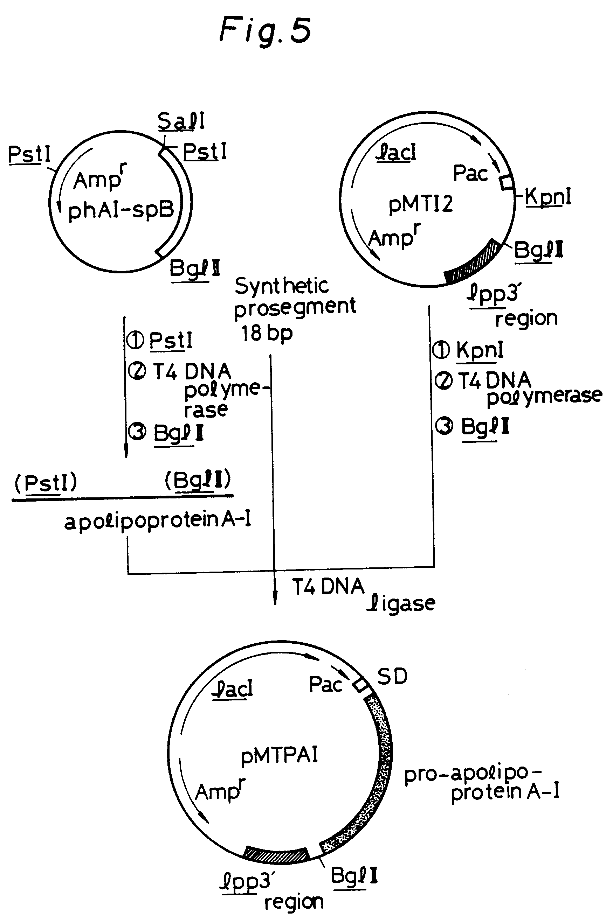

Fig.5 represents a scheme of the construction of an expression plasmid pMTPAI for producing pro-apolipoprotein A-I.

### DETAILED DESCRIPTION OF THE INVENTION

DNA fragments according to the present invention may be prepared in the following manner.

Human-derived specimens such as liver sections, small intestine epidermal cells, blood macrophages and kidney sections are homogenized in the presence of guanidinyl thiocyanate and total RNA is separated by CsCl equilibrium density gradient ultracentrifugation (Chirgwin et al., Biochemistry, 18, 5294-5299, 1979). The total RNA is then purified by conventional oligo(dT) cellulose column chromatography so as to isolate poly(A)-containing RNAs which are served as mRNA materials.

The mRNAs thus obtained are treated by the method described by Okayama and Berg in Molecular and Cellular Biology, 2, 161-170, (1982) to prepare cDNA library. A vector primer and an oligo(dG)-tailed linker are obtained from a hybrid plasmid of pBR322 and SV40. The vector primer and mRNA materials are used to synthesize cDNAs in the presence of reverse transcriptase. The cDNAs are digested with HindIII and then cyclized together with the above linker. The mRNAs portions are replaced by DNAs to give cDNA fragment-containing plasmids.

The plasmids thus prepared are used to transform Escherichia coli (E.coli) or the other microorganism in a conventional manner. The resulting ampicillin resistant transformants are screened by using, as a probe, a synthetic oligonucleotides containing at least the base sequence corresponding to the amino acids sequence at positions 108-112 of apolipoprotein A-I (Trp-Gln-Glu-Glu-Met). Thus, the clones carrying a base sequence complementary to said base sequence are selected. Plasmids from such clones are treated with an appropriate restriction enzyme to select clones containing a plasmid into which the longest cDNA has been inserted.

The base sequence of the above cDNA fragment is determined by the method of Maxam and Gilbert (Methods in Enzymology, 65, 499-560, 1980).

The DNA fragments of the present invention, which are to be cloned in an expression vector, contain the DNA sequence encoding pro-apolipoprotein A-I or pro-apolipoprotein A-I-like proteins.

The DNA sequence encoding pro-apolipoprotein A-I has DNA sequence encoding a propeptide of 6 amino acids, which is linked upstream from (5′ to) the DNA sequence encoding the mature apolipoprotein A-I (Proc. Natl. Acad. Sci. USA, 81, 66-70, 1984).

Any DNA fragment in which a portion of the DNA sequence encoding pro-apolipoprotein A-I has been deleted or substituted by other base(s), or one or more bases has been added thereto may be advantageously used in the present invention as long as the DNA sequence thus modified encodes pro-apolipoprotein A-I-like proteins.

A naturally-occurring propeptide of apolipoprotein A-I is encoded by the following base sequence:

5'- CGG CAT TTC TGG CAG CAA -3'
3'- GCC GTA AAG ACC GTC GTT -5'.

In accordance with the invention, the following base sequence: 
5'- CGA CAT TTT TGG CAA CAA -3'
3'- GCT GTA AAA ACC GTT GTT -5';
which is partly modified may be advantageously used because it may enhance a rate of expression.

The expression vectors of the present ivnetion may contain a procaryotic promoter which is located in order to transcriptionally control a gene for pro-apolipoprotein A-I or pro-apolipoprotein A-I-like proteins (referred to hereinafter as a pro-apolipoprotein A-I structural gene).

Preferred promoters include those which can be derived from E coli such as trp, lac, lac UV5 and rec A, P_{L}P_{R} from λ phage those of gene 25 or 26 from T5 phage, those from Bacillus subtilis such as DHFR gene promoter, a strong and controllable promoter such as tac (Proc. Natl. Acad. Sci., 81, 21, 1983) and pac being more preferred.

The expression vectors according to the present invention may further contain a repressor gene which may act as a regulator gene for promoters, a ribosomal binding site for initiating translation and a terminator sequence such as lpp3′region for terminating transcription, which are optionally combined in order to increase the expression efficiency of the resulting expression vectors. The pro-apolipoprotein A-I structural gene may be preferably cloned at the site between the ribosomal binding site and the terminator sequence. The expression vectors of the present invention may also contain a selective marker, for example, a resistance gene to antibiotics such as ampicillin so that the resulting transformants may be easily selected.

The expression vectors according to the present may be introduced into E coli in a conventional CaCl₂ method described by Mandel et al. in Journal of Molecular Biology, 53, 154, 1970. The transformants thus obtained are cultured in a culture medium such as L broth and M9 Medium. Upon addition to the culture medium of isopropyl-β-D-thiogalactopyranoside as an inducer of the promoters, the expression of pro-apolipoprotein A-I or pro-apolipoprotein A-I-like proteins is induced leading to the production thereof in the host cells.

The resulting proteins may be purified by disrupting cells, treating the resulting crude cell extracts with lipids such as dimyristoyl phosphatidylcholine (DMPC) to form a complex, fractionating the resulting complex by KBr density gradient centrifugation and collecting a cushion with density ρ of 1.08 -1.12.

According to the present invention, human pro-apolipoprotein A-I or human pro-apolipoprotein A-I-like proteins may be efficiently produced. The proteins obtained in such ways may be used as antigens to prepare antisera by an usual method. The antisera can be employed in the detection of the presence of pro-apolipoprotein A-I.

Furthermore, human pro-apolipoprotein A-I or human pro-apolipoprotein A-I-like proteins prepared in the present invention may be converted into a mature type in blood stream without any pre-procedure such as removal of its propeptide prior to the administration thereof, so that they can be used as an anti-hyperlipidemia or anti-arteriosclerosis agent just like the mature human apolipoprotein A-I.

### DESCRIPTION OF THE PREFERRED EMBODIMENTS

The invention will be more fully illustrated with reference to the following examples, which may not be construed as limiting the scope of the present invention.

### EXAMPLE 1:

### PREPARATION OF EXPRESSION PLASMIDS:

### Modification of cDNA sequence for human pro-apolipoprotein A-I by site-specific mutation:

### Mutation at N terminus

phAI-6 (5 µg) obtained by the method described in the specification of Japanese Patent Application Laying Open (KOKAI) No. 61-96998 was digested with 10 u of PstI at 37°C for 2 hours in 100 µl of a buffer solution containing 10 mM Tris-HCl (pH 7.5), 100 mM NaCl and 6 mM MgCl₂ (referred to hereinafter as "buffer solution H"). After inactivation of the enzyme by heating at 75°C for 15 minutes, the reaction mixture was dialyzed against water and dried. To the resulting dry powder, a buffer solution containing 33 mM Tris-HCl (pH 7.9), 66 mM potassium acetate, 10 mM magnesium acetate and 0.5 mM dithiothreitol (DTT) (referred to hereinafter as "T4 DNA polymerase buffer solution") was added and four kinds of deoxynucleotide triphosphate (dNTP) were further added to a final concentration of 2 mM each to yield a mixture with total volume of 40 µl. The 3′ protruding ends were digested with T4 DNA polymerase (4u). After inactivation of the enzyme by heating at 70°C for 10 minutes, the reaction mixture was dialyzed against water and dried. The resulting fragments, designated Fragment I, were stored before use in an aqueous solution (50 µl).

On the other hand, 20 µg of phAI-6 was digested with EcoRI and SacI (20 u each) at 37°C for 2 hours in 100 µl of buffer solution H. The digested fragments were separated electrophoretically on 5% acrylamide gel (89 mM Tris-HCl, 89 mM boric acid, 2 mM EDTA; 10 V/cm, 1.5 hours). After electrophoresis, the gel was stained with 0.05% ethidium bromide aqueous solution and a gel band containing larger fragments was excised from the gel under the irradiation of ultraviolet at 340 nm. The excised gel band was crushed using a glass rod, suspended in 4 ml of a DNA-extracting buffer solution (0.5 M ammonium acetate, 10 mM magnesium acetate, 1 mM EDTA and 0.1% sodium lauryl sulfate) and then allowed to stand overnight at 37°C. After removal of larger gel tips by centrifugation at 10,000 r.p.m. for 15 minutes and smaller ones by glass filters, the extracted DNA fragments were purified by repeating precipitation in ethanol three times. The purified DNA fragments, designated Fragment II, were stored in an aqueous solution (200 µl).

The following DNA fragment consisting of 41 bp was synthesized as a primer using a DNA synthesizer (Applied Biosystem MODEL 380A):

Synthesized primer:          ┌─SalI─┐┌─PstI─┐
5'- GC CAG CCT CGG CAT TGT CGA CCT GCA GAC GAA CCC CCC CAG -3';
Original base sequence:
5'- GC CAG CCT CGG CAT TTC TGG CAG CAA GAC GAA CCC CCC CAG -3'.

The resulting primer was phosphorylated at 5′ end by 20 u of T4 polynucleotide kinase in 10 µl of a kinase buffer solution (50 mM Tris-HCl (pH 8.0), 10 mM MgCl₂ and 5 mM DTT).

To a mixture of 0.05 pmol of Fragment I, 0.05 pmol of Fragment II and 45 pmol of the 5′-phosphorylated primer, 12 µl of a ligase buffer solution (0.5 M NaCl, 32.5 mM Tris-HCl (pH 7.5), 40 mM MgCl₂ and 5 mM β-mercaptoethanol) and 5x conc. polymerase were added to give a mixture with total volume of 34.8 µl. The resultimg mixture was boiled at 100°C for 3 minutes immediately followed by a successive incubation at 30°C for 30 minutes, at 4°C for 30 minutes and on ice for 10 minutes so as to form heteroduplexes.

The heteroduplexes (11.6 µl) were mixed with 4 µl of four kinds of dNTP (2.5 mM each), 2 µl of 10 mM ATP, 2 u of Klenow enzyme and 0.5 u of T4 DNA ligase and the resulting mixture (20 µl) was incubated overnight at 16°C for cyclization of the heteroduplexes.

The cyclized plasmid was used to transform E.coli HB101. The transformants were cultured and the same plasmid was obtained therefrom in a conventional manner, which was revealed to be a mutant plasmid comprising two PstI sites.

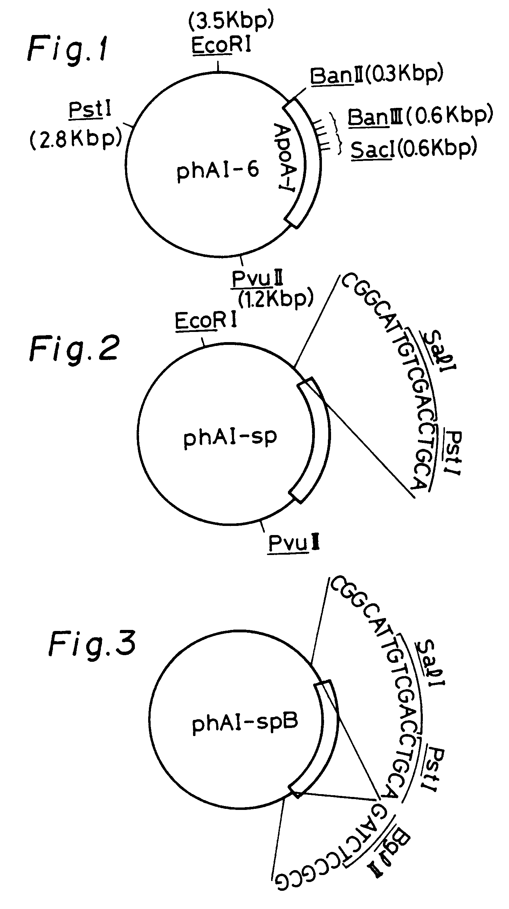

Since the mutant plasmid prepared as above was considered to be contaminated with a starting plasmid, i.e., a wild-type plasmid, it was subjected to a further purification through transformation thereby. Thus, plasmid phAI-sp was obtained, which is shown in FIG.2.

### Mutation at C-terminus:

phAI-sp (5 µg) was digested with 10 u of PstI at 37°C for 2 hours in 100 µl of buffer solution H. After inactivation of the enzyme by heating at 75°C for 15 minutes, the reaction mixture was dialyzed against water and dried. To the resulting dry powder, T4 DNA polymerase buffer solution was added and four kinds of dNTP were further added to a final concentration of 2 mM each to yield a mixture with total volume of 40 µl. The 3′ protruding ends were digested with T4 DNA polymerase (4u). After inactivation of the enzyme by heating at 70°C for 10 minutes, the reaction mixture was dialyzed against water and dried. The resulting fragments, designated Fragment I', were stored before use in an aqueous solution (50 µl).

On the other hand, 20 µg of phAI-sp was digested with PvuII and SacI (5 u each) at 37°C for 2 hours in 100 µl of buffer solution H. The digested fragments were separated electrophoretically on 5% acrylamide gel (89 mM Tris-HCl, 89 mM boric acid, 2 mM EDTA; 10 V/cm, 1.5 hours). After electrophoresis, the gel was stained with 0.05% ethidium bromide aqueous solution and a gel band containing larger fragments was excised from the gel under the irradiation of ultraviolet at 340 nm. The excised gel band was crushed using a glass rod, suspended in 4 ml of the DNA-extracting buffer solution and then allowed to stand at 37°C overnight. After removal of larger gel tips by centrifugation at 10,000 r.p.m. for 15 minutes and smaller ones by glass filters, the extracted DNA fragments were purified by repeating precipitation in ethanol three times. The purified DNA fragments, designated Fragment II', were stored in an aqueous solution (200 µl).

The following DNA fragment consisting of 36 bp was synthesized as a primer using said DNA synthesizer:

Synthesized primer:                    BglII
5'- G CTC AAC ACC CAG TGA GAT CTC CGC GCC GCC CCC CT -3';
Original base sequence:
5'- G CTC AAC ACC CAG TGA GGC GCC CGC GCC GCC CCC CT -3'.

The resulting primer (150 pmol) was 5′-phosphorylated by 20 u of T4 polynucleotide kinase in 10 µl of the kinase buffer solution.

Plasmid phAI-spB was obtained in the same manner as in Mutation at N-terminus except that Fragment I′, Fragment II′ and the 5′-phosphorylated primer prepared above were used. Plasmid phAI-spB is shown in FIG.3.

### EXAMPLE 2:

### CONSTRUCTION OF EXPRESSION PLASMIDS FOR HUMAN PRO-APOLIPOPROTEIN A I:

A DNA fragment consisting of the following base sequence encoding a prosegment was synthesized by using the above DNA synthesizer:

(Arg His Phe Trp Gln Gln)
5'- CGA CAT TTT TGG CAA CAA -3'
3'- GCT GTA AAA ACC GTT GTT -5'.

The resulting DNA fragment (45 pmol) was 5′-phosphorylated by 20 u of T4 polynucleotide kinase in 10 µl of the kinase buffer solution at 37°C for one hour.

On the other hand, plasmid phAI-spB (10 µg) was digested with 20 u of PstI for 2 hours in buffer solution H. After inactivation of the enzyme by heating at 75°C for 10 minutes, the reaction mixture was dialyzed against water, desalted and dried.

The dry powder was dissolved in 50 µl of the T4 DNA polymerase buffer solution and treated with 10 u of T4 DNA polymerase at 37°C for 30 minutes so as to delete the 3′ protruding ends. Thus, the base sequence ligated to the 5′ end of the DNA sequence encoding apolipoprotein A-I was removed out. The enzyme was inactivated by heating at 70°C for 10 minutes. 5.5 µl of 10 x conc. buffer solution H (0.1 M Tris-HCl, 1 M NaCl and 60 mM MgCl₂) was mixed with the resulting solution followed by digestion with 10 u of BglII. The digested fragments were separated electrophoretically on 5% acrylamide gel under the same condition as the above to isolate a DNA fragment of about 736 bp, which was designated Fragment MAI.

Plasmid pMTI2 (2 µg) prepared in REFERENCE EXAMPLE described infra was digested with 5 u of KpnI at 37°C for 2 hours in 20 µl of a buffer solution (10 mM Tris-HCl (pH7.5), 6 mM MgCl₂). To this reaction mixture was added 2 µl of 10 x conc.T4 DNA polymerase buffer solution. Upon addition of four kinds of dNTP to a final concentration of 1 mM each, the 3′ protruding ends were deleted by digestion with T4 DNA polymerase at 37°C for 30 minutes followed by inactivation of the enzyme by heating at 70°C for 10 minutes.

This reaction mixture was mixed with 2.5 µl of 10 x conc. buffer solution H and treated with 4 u of BglII at 37°C for 2 hours to cleave the DNA fragments. Proteins in the resulting solution were extracted in a saturated phenol aqueous solution and a supernatant (a water layer) was separated from an organic layer. The water layer was washed with ether to remove phenol therefrom, dialyzed against water, desalted, dried and dissolved again in 10 µl of water.

The synthesized prosegment (4.5 pmol), 1 µg of Fragment MAI, 0.5 µg of the DNA fragment prepared from pMTI2 and 1 µl of 0.1 M ATP were added into 10 µl of a buffer solution (10 mM Tris-HCl, 6 mM MgCl₂, 1 mM DTT) and these fragments were ligated to one another by incubation in the presence of 10 u of T4 DNA ligase at 4°C for 16 hours. The resulting plasmids ( 3 µl) were then used to transform E. coli JM109 competent cells (TAKARA SHUZO CO. LTD.) in accordance with a conventional method.

Screening for the transformants harboring the desired plasmid was carried out by an immunoscreening method using anti-human apolipoprotein A-I antibodies. Thus, the transformants obtained were streak-cultured on two agar plates, one of which was served as a master plate and the other containing 2 mM IPTG was covered with a nitrocellulose filter (referred to hereinafter as "IPTG plate"). The two plates were incubated overnight at 30°C.

After the incubation, the master plate was stored. On the other hand, the nitrocellulose filter was teared off and sandwiched between two filter papers infiltrated with 5% SDS solution followed by heating at 100°C for 20 minutes. The nitrocellulose filter was then sandwiched between two pieces of sponge infiltrated with a buffer solution (14.4 g/L of glycine and 3.3 g/L of Tris) to remove SDS therefrom by electrophoresis in said buffer solution under 8 V/cm at 4°C for 30 minutes. The resulting nitrocellulose filter was blocked in TBS buffer solution containing 3% gelatin (3% GTBS, 10 mM Tris-HCl, pH7.5) at 37°C for one hour.

The blocked nitrocellulose filter was incubated overnight at a room temperature with anti-human apolipoprotein A-I rabbit antibodies diluted 1,000 times in TBS buffer solution containing 1% gelatin (1% GTBS). The nitrocellulose filter was then washed twice with TBS buffer solution containing 0.05% Tween 20 (TTBS) at a room temperature for 15 minutes each. The washed nitrocellulose filter was further incubated with anti-rabbit IgG antibodies labelled with horseradish peroxydase (Bio-Rad) diluted 1,000 times in 1% GTBS at a room temperature for one hour. The same washing procedure using TTBS as the above was repeated two times. A substrate solution was prepared by mixing 12 mg of a color-developing reagent of 4-chloro-1-naphthol (Bio-Rad), 4 ml of methanol, 12 µl of 30% H₂O₂ and 20 ml of TBS buffer solution. The (hybridized) nitrocellulose filter was subjected to color development in the substrate solution for about 10 minutes. The colonies on the master plate, which corresponded to the dots observed on the nitrocellulose filter, were selected therefrom. Thus, the cells producing pro-apolipoprotein A-I were screened and selected.

### EXAMPLE 3:

### PRODUCTION AND PURIFICATION OF HUMAN PRO APOLIPOPROTEIN A-I:

The cells producing pro-apolipoprotein A-I were subjected to shaking culture at 30°C for 2 hours in L broth containing 10 g/l of bactotrypton, 5 g/l of yeast extract and 10 g/l of NaCl, supplemented with ampicillin (50 mg/l). Upon addition of IPTG to a final concentration of 2 mM, the shaking culture was continued at 30°C for 3 hours. The cells were harvested by centrifugation at 6,500 r.p.m. for 10 minutes. The resulting cell pellet was resuspended in a solution (10 mM Tris-HCl (pH 7.5), 0.1 mM EDTA) of 10 times the amount of the cell pellet, heated in boiling water for 5 minutes and disrupted through sonication for about one minute to obtain extracts of pro-apolipoprotein A-I. Cell debris was removed by centrifugation at 10,000 r.p.m. for 20 minutes.

L-α-phosphatidylcholine dimyristoyl (DMPC) was added to the resulting supernatant in the ratio of 3 mg thereof per one mg of pro-apolipoprotein A-I. Sonication of the mixture for 45 minutes resulted in a formation of a pro-apolipoprotein A-I-DMPC complexes. Following dialysis overnight against PBS containing 10 mM potassium phosphate (pH 7.5) and 0.5 % NaCl, the sonicated mixture was placed on density gradient solution of KBr-PBS (ρ is 1.01 - 1.21) and subjected to ultracentrifugation at 50,000 r.p.m. for 48 hours.

The pro-apolipoprotein A-I-DMPC complexes were fractionated in a fraction of about 1.1 ρ, which fraction was further purified by reverse phase HPLC so as to isolate peak fractions corresponding to pro-apolipoprotein A-I.

Determination of the amino acid composition and N-terminal amino acid sequence of the purified pro-apolipoprotein A-I shown below confirmed its authenticity.

### Amino acid composition:

| amino acid | total amount (nmol) | content found (mol/mol) | content deduced from the base sequence (mol/mol) |
|---|---|---|---|
| Asp | 2.95 | 20.5 | 21 |
| Thr ^{a)} | 1.30 | 9.0 | 10 |
| Ser ^{b)} | 1.91 | 13.3 | 15 |
| Glu | 6.57 | 45.7 | 46 |
| Pro | 1.65 | 11.5 | 10 |
| Gly | 1.67 | 11.6 | 12 |
| Ala | 2.36 | 16.4 | 19 |
| Cys | | 0 | 0 |
| Val ^{b)} | 1.87 | 13.0 | 13 |
| Met | 0.49 | 3.4 | 4 |
| Ile ^{b)} | 0.12 | 0.9 | 0 |
| Leu ^{b)} | 5.17 | 35.9 | 37 |
| Tyr | 0.91 | 6.3 | 7 |
| Phe | 0.97 | 6.7 | 7 |
| Lys | 3.02 | 21.0 | 21 |
| His | 0.71 | 5.0 | 6 |
| Arg | 2.17 | 15.1 | 17 |
| Trp | 0.28 | 2.0 | 5 |
| hydrolysis by methanesulfonic acid at 110°C for 24 or 48 hours | | | |

| | | | |
|---|---|---|---|
| a) after calibration of the data obtained at hour 24 or 48 to 0 hour | | | |
| b) the data obtained at hour 48 | | | |

### N terminal amino acid sequence:

1                                    10
Met-Arg-His-Phe-Trp-Gln-Gln-Asp-Glu-Pro-Pro-Gln-Ser-Pro-Trp-Asp-
        19
Arg-Val-Lys

### REFERENCE EXAMPLE:

### CONSTRUCTION OF PLASMID pMTI2

### Construction of hybrid promoter:

The following four DNA fragments were prepared by means of the above DNA synthesizer:
1. A DNA fragment containing -35 region of T5 phase p25 promoter: 
5'-AATTCAAAAATTTATTTGCTTTCAGGAAAATTTTT
CT-3'
2. A DNA fragment complementay to the fragment 1.: 
5'-ATACAGAAAAATTTTCCTGAAAGCAAATAAATTTT
TG-3'
3. A DNA fragment containing -10 region, an operator region and a starting site of RNA biosynthesis of lac UV5 promoter: 
5'-GTATAATGTGTGGAATTGTGAGCGGATAACAATTT
CGGATCCA-3'
4. A DNA fragment complementary to the fragment 3.:

5'-AGCTTGGATCCGAAATTGTTATCCGCTCACAATTC
CACACATT-3'

EcoR I

-35
① 5' AATTCAAAAATTTATTTGCTTTCAGGAAAATTTTTCT3'
3' GTTTTTAAATAAACGAAAGTCCTTTTAAAAAGACATA5' ②

-10 --------Operator-------- BamH I Hind III
③ 5' GTATAATGTGTGGAATTGTGAGCGGATAACAATTTCGGATCCA3'
3' TTACACACCTTAACACTCGCCTATTGTTAAAGCCTAGGTT
CGA5' ④

Following removal of protecting groups by treatment with 28% ammonia aqueous solution (2 ml) at 55°C for 6 hours, 100 µg of each synthesized DNA fragment was purified by electrophoresis on 17% acrylamide/7 M urea gel to give 10 µg of each DNA fragment. The purified DNA fragment (1 µg each) was phosphorylated at 5′ end by incubation with 10 µl of a buffer solution containing 1 u of T4 polynucleotide kinase, 10 mM MgCl₂ and 10 mM Tris-HCl (pH 7.6) at 37°C for one hour. After addition of NaCl to a final concentration of 50 mM, the phosphorylated DNA fragments 1 and 2, and the phosphorylated DNA fragments 3 and 4 (1 µg each) were mixed with each other, respectively, heated at 100°C for 3 minutes, allowed to cool for annealing to obtain 2 µg of double stranded DNA fragments of 37 bp and 43 bp, respectively.

The resulting two double stranded DNA fragments (0.5 µg each) were ligated into 1 µg of plasmid pBR 322 digested with EcoRI and HindIII using T4 DNA ligase (1 u) at 4°C for 16 hours in 10 µl of a buffer solution containing 10 mM Tris-HCl (pH 7.5), 6 mM MgCl₂, 10 mM ATP and 1 mM DTT.

E.coli HB101 competent cells were prepared in a conventional manner. Thus, E. coli HB101 cells were collected in a logarithmic phase, suspended in one thirds volume of 0.1 M CaCl₂, allowed to stand at 0°C for 30 minutes, recollected and resuspended in 1/100 volume of 0.1 M CaCl₂. E. coli HB101 competent cells thus prepared were mixed with the solution containing the constructed plasmids, supplemented with 100 mM CaCl₂ and incubated successively at 0°C for 10 minutes and at 37°C for 5 minutes. The resulting transformants were easily selected by subjecting to culture in a medium containing 20 µg/ml of tetracycline. Thus, plasmid pBRpac having pac promoter activity has been cloned.

The base sequence of pac promoter was determined by a conventional dideoxy method described in Anal. Biochem. Vol. 152,232.

### Insertion of transcription termination factor:

Plasmid pBRpac (4.4 Kbp) in an amount of 1 µg was digested with 1 u each of BamHI and PvuII at 37°C for 2 hours in 10 µl of buffer solution H. The product was precipitated in ethanol followed by evaporation to dryness for reservation.

On the other hand, 5 µg of plasmid pINIIIAl described in The EMBO J., Vol.3, 10, 2437, 1984 was digested with 5 u of KpnI at 37°C for 2 hours in 50 µl of a buffer solution containing 10 mM Tris-HCl (pH 7.5) and 6 mM MgCl₂. To this was added 5.5 µl of a buffer solution containing 660 mM potassium acetate, 100 mM magnesium acetate and 330 mM Tris-HCl (pH 7.9) to delete the 3′ protruding ends in the presence of 10 u of T4 DNA polymerase at 37°C for 15 minutes. The enzyme was inactivated by heating at 70°C for 10 minutes. To this reaction mixure was added 6.0 µl of 10 x conc. buffer solution H and the plasmid was further subjected to digestion with 5 u of BamHI at 37°C for 2 hours. The resulting mixture was treated with a saturated phenol aqueous solution to remove proteins and ethanol precipitation was repeated three times.

Thus, a DNA fragment containing lpp3′ region ( -500 bp) which comprises a transcription termination factor was purified with a yield of 0.5 µg. The resulting DNA fragment was ligated into the digested pBRpac in the presence of 1 u of T4 DNA ligase in 10 µl of a buffer solution containing 10 mM Tris-HCl (pH 7.5), 10 mM MgCl₂, 10 mM ATP and 1 mM DTT (referred to hereinafter as "buffer solution A") at 4°C for 16 hours. The resulting plasmid was designated pPac.

Plasmid pPac could be stably replicate in JM 105 or JM 109, which is a lacI^{q} strain. Thus, 1 mg of pPac was obtained by transforming JM 105 strain in the same manner as the above, culturing the transformants in 1 l of a minimum medium supplemented by 20 mg/ml of ampicillin and purifying in a conventional manner.

### Introduction of multi-linker:

A multi-linker containing SD sequence and an initiation Met codon was synthesized using Model 380A. The synthesized multi-linker was subjected to deprotection in the same way as the above followed by purification on 17% acrylamide/7 M urea gel to obtain 10 µg of a purified DNA.

On the other hand, plasmid pPac (1 µg) was digested with 1 u of BamHI in 10 µl of buffer solution H at 37°C for 2 hours, precipitated with ethanol and evaporated to dryness.

The multi-linker (1 µg) and the digested pPac were ligated to each other in the presence of 1u of T4 DNA ligase at 4°C for 16 hours. The resulting plasmid was designated pMT2.

### Introduction of lac repressor gene:

Ten µg of plasmid pMC 9 described in Proc. Natl. Acad. Sci. USA, 80, 3015, (1983) was digested with 10 u of EcoRI at 37°C for 2 hours in 100 µl of a buffer solution containig 100 mM Tris-HCl (pH 7.5), 10 mM NaCl and 6 mM MgCl₂ followed by ethanol-precipitaion and evaporation to dryness to obtain 2.5 µg of 1.7 Kbp DNA fragment comprising a repressor gene lacI.

On the other hand, 1 µg of plasmid pMT2 was digested with 1 u of EcoRI at 37°C for 2 hours in 10 µl of the buffer solution containig 100 mM Tris-HCl (pH 7.5), 10 mM NaCl and 6 mM MgCl₂ followed by ethanol-precipitaion and evaporation to dryness.

The 1.7 Kbp DNA fragment (1 µg) and the digested pMT2 were ligated to each other in the presence of 1 u of T4 DNA ligase in 20 µl of buffer solution A to yield plasmid pMTI2.

## Claims

1. A process for the production of human pro-apolipoprotein A-I or human pro-apolipoprotein A-I-like substances having physiological properties equivalent to those of human pro-apolipoprotein A-I, comprising introducing by transformation into host cells an expression vector containing DNA sequence encoding said human pro-apolipoprotein A-I or human pro-apolipoprotein A-I-like substances, culturing the transformants and collecting the proteins produced thereby, characterized in that the DNA sequence encoding the pro-portion of the human pro-apolipoprotein A-I or human pro-apolipoprotein A-I-like substances comprises the sequence :

5' - CGA CAT TTT TGG CAA CAA - 3'
3' - GCT GTA AAA ACC GTT GTT - 5'.

## Patentansprüche

1. Verfahren zur Herstellung von menschlichem Pro-apolipoprotein A-I oder menschlichen pro-apolipoprotein-A-I-ähnlichen Substanzen mit physiologischen Eigenschaften, die denen von menschlichem Pro-apolipoprotein A-I äquivalent sind, umfassend eine durch Transformation vorgenommene Einfuhrung eines Expressionsvektors mit einem Gehalt an einer DNA-Sequenz, die für menschliches Pro-apolipoprotein A-I oder menschliche pro-apolipoprotein A-I-ähnliche Substanzen codiert, in Wirtszellen, das Züchten der transformierten Wirtszellen und das Gewinnen der dabei gebildeten Proteine, dadurch gekennzeichnet, daß die DNA-Sequenz, die für den Pro-bereich von menschlichem Pro-apolipoprotein A-I oder menschlichen pro-apolipoprotein-ähnlichen Substanzen codiert, folgende Sequenz umfaßt:

5'-CGA CAT TTT TGG CAA CAA-3'
3'-GCT GTA AAA ACC GTT GTT-5'.

## Revendications

1. Procédé de production de la pro-apolipoprotéine A-I humaine ou de substances semblables à la pro-apolipoprotéine A-I humaine et ayant des propriétés équivalentes à celles de la pro-apolipoprotéine A-I humaine, comprenant l'introduction, par transformation, dans des cellules hôtes, d'un vecteur d'expression contenant une séquence d'ADN codant pour ladite pro-apolipoprotéine A-I humaine, ou pour les substances semblables à la pro-apolipoprotéine A-I humaine, le développement de la culture des transformants, et la récupération des protéines ainsi produites, caractérise en ce que la séquence d'ADN codant pour la partie "pro" de la pro-apolipoprotéine A-I humaine ou des substances semblables à la pro-apolipoprotéine A-I humaine comprend la séquence :
